# EUROPEAN PATENT SPECIFICATION

(11) **EP 2 361 071 B1**
(45) Date of publication and mention of the grant of the patent: **05.08.2015**
(21) Application number: 09833020.2
(22) Date of filing: 15.11.2009
(51) Int. Cl.: A61F 13/42, A61F 13/53, A61F 13/505

(54) **PHYSICAL SENSATION ABSORBENT ARTICLE**
ARTIKEL ZUR ABSORPTION VON TASTEMPFINDUNGEN
ARTICLE ABSORBANT À SENSATION PHYSIQUE

(30) Priority: 15.12.2008 US 316651
(43) Date of publication of application: 31.08.2011
(73) Proprietor: Kimberly-Clark Worldwide, Inc., Neenah, Wisconsin 54956 (US)
(72) Inventor: LONG, Andrew, Mark, Appleton Wisconsin 54915 (US); YU, Lisha, Appleton Wisconsin 54911 (US); WEBER, Shirlee, Ann, Neenah Wisconsin 54956 (US)
(74) Representative: Davies, Christopher Robert
(86) International application number: PCT/IB2009/055076
(87) International publication number: WO 2010/070478

(56) References cited:
- WO-A1-00/00082
- WO-A1-96/19172
- KR-A- 20060 027 800
- US-A- 5 702 376
- US-A1- 2002 169 427
- US-A1- 2003 014 025
- US-A1- 2003 100 872

## Description

### BACKGROUND

The present disclosure relates to absorbent articles that include a physical sensation member. More specifically, the disclosure relates to an absorbent article such as training pants that provides the wearer with a noticeable physical sensation upon urination.

Absorbent articles such as disposable diapers and training pants are useful to absorb and contain body wastes. These products have developed to the extent that urine is quickly drawn and retained away from the wearer's skin so that the wearer remains relatively dry and comfortable. Although this improved performance enhances wearer dryness and comfort, it can reduce the wearer's ability to notice or recognize when urination occurs, especially if the wearer's attention is distracted by an activity. This is not conducive to toilet training because an important step in the early stages of toilet training is the ability to recognize when urination occurs. In an attempt to enhance a child's recognition of when urination occurs, training pants have been designed with temperature change members that provide a temperature change sensation upon urination.

Unfortunately, in certain circumstances, such temperature change members might not be completely satisfactory. For example, the element providing the temperature change sensation might not be in contact with the wearer's skin, thus limiting the effectiveness of the sensation.

Thus, there is a need for an absorbent article with a physical sensation member that is capable of more effectively providing a physical sensation to the wearer.

US2002/0169427 discloses an absorbent article according to the preamble of claim 1.

### SUMMARY

In accordance with the present invention, there is provided an absorbent article as set forth in claim 1.

The purposes and features of the present disclosure will be set forth in the description that follows. Additional features of the disclosure might be realized and attained by the product and processes particularly pointed out in the written description and claims hereof, as well as from the appended drawings.

It is to be understood that both the foregoing general description and the following detailed description are exemplary and are intended to provide further explanation of the disclosure. The accompanying drawings, which are incorporated in and constitute part of this specification, are included to illustrate and provide a further understanding of the disclosure.

### BRIEF DESCRIPTION OF THE DRAWINGS

The present disclosure will be more fully understood, and further features will become apparent, when reference is made to the following detailed description and the accompanying drawings. The drawings are merely representative and are not intended to limit the scope of the claims.
Figure 1 representatively illustrates a side view of a pair of training pants with a mechanical fastening system of the pants shown fastened on one side of the training pants and unfastened on the other side of the training pants;
Figure 2 representatively illustrates a plan view of the training pants of Figure 1 in an unfastened, stretched and laid flat condition, and showing the surface of the training pants that faces away from the wearer;
Figure 3 representatively illustrates a plan view similar to Figure 2, but showing the surface of the training pants that faces the wearer when worn, and with portions cut away to show underlying features;
Figure 4 representatively illustrates a schematic cross-section view of a particular aspect of the physical sensation member of the present disclosure; and
Figure 5 representatively illustrates a schematic cross-section view of another physical sensation. This example *per se* is not within the scope of the present invention.

Repeat use of reference characters in the present specification and drawings is intended to represent the same or analogous features or elements of the present disclosure. The drawings are representational and are not necessarily drawn to scale. Certain proportions thereof might be exaggerated, while others might be minimized.

### DEFINITIONS

Within the context of this specification, each term or phrase below includes the following meaning or meanings:
"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.
"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.
"Coform" refers to a blend of meltblown fibers and absorbent fibers such as cellulosic fibers that can be formed by air forming a meltblown polymer material while simultaneously blowing air-suspended fibers into the stream of meltblown fibers. The coform material can also include other materials, such as superabsorbent materials. The meltblown fibers and absorbent fibers are collected on a forming surface, such as provided by a foraminous belt. The forming surface can include a gas-pervious material that has been placed onto the forming surface.
"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.
"Disposable" refers to articles that are designed to be discarded after a limited use rather than being laundered or otherwise restored for reuse.
The terms "disposed on," "disposed along," "disposed with," or "disposed toward" and variations thereof are intended to mean that one element can be integral with another element, or that one element can be a separate structure bonded to or placed with or placed near another element.
"Elastic," "elasticized," "elasticity," and "elastomeric" mean that property of a material or composite by virtue of which it tends to recover its original size and shape after removal of a force causing a deformation. Suitably, an elastic material or composite can be elongated by at least 25 percent (to 125 percent) of its relaxed length and will recover, upon release of the applied force, at least 40 percent of its elongation.
"Extensible" refers to a material or composite that is capable of extension or deformation without breaking, but does not substantially recover its original size and shape after removal of a force causing the extension or deformation. Suitably, an extensible material or composite can be elongated by at least 25 percent (to 125 percent) of its relaxed length.
"Fiber" refers to a continuous or discontinuous member having a high ratio of length to diameter or width. Thus, a fiber can be a filament, a thread, a strand, a yarn, or any other member or combination of these members.
"Hydrophilic" describes fibers or the surfaces of fibers that are wetted by aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with this system, fibers having contact angles less than 90 degrees are designated "wettable" or hydrophilic, and fibers having contact angles greater than 90 degrees are designated "nonwettable" or hydrophobic.
"Layer" when used in the singular can have the dual meaning of a single element or a plurality of elements.
"Liquid impermeable," when used in describing a layer or multi-layer laminate means that liquid, such as urine, will not pass through the layer or laminate, under ordinary use conditions, in a direction generally perpendicular to the plane of the layer or laminate at the point of liquid contact.
"Liquid permeable" refers to any material that is not liquid impermeable.
"Meltblown" refers to fibers formed by extruding a molten thermoplastic material through a plurality of fine, usually circular, die capillaries as molten threads or filaments into converging high velocity gas (e.g., air) streams, generally heated, which attenuate the filaments of molten thermoplastic material to reduce their diameters. Thereafter, the meltblown fibers are carried by the high velocity gas stream and are deposited on a collecting surface to form a web of randomly dispersed meltblown fibers. Such a process is disclosed, for example, in U.S. Patent 3,849,241 to Butin et al. Meltblowing processes can be used to make fibers of various dimensions, including macrofibers (with average diameters from about 40 to about 100 microns), textile-type fibers (with average diameters between about 10 and 40 microns), and microfibers (with average diameters less than about 10 microns). Meltblowing processes are particularly suited to making microfibers, including ultra-fine microfibers (with an average diameter of about 3 microns or less). A description of an exemplary process of making ultra-fine microfibers can be found in, for example, U.S. Patent No. 5,213,881 to Timmons, et al. Meltblown fibers can be continuous or discontinuous and are generally self bonding when deposited onto a collecting surface.
"Member" when used in the singular can have the dual meaning of a single element or a plurality of elements.
"Nonwoven" and "nonwoven web" refer to materials and webs of material that are formed without the aid of a textile weaving or knitting process. For example, nonwoven materials, fabrics or webs have been formed from many processes such as, for example, meltblowing processes, spunbonding processes, air laying processes, and bonded carded web processes.
"Stretchable" means that a material can be stretched, without breaking, by at least 25 percent (to 125 percent of its initial (unstretched) length) in at least one direction. Elastic materials and extensible materials are each stretchable materials.
"Superabsorbent material" refers to a water-swellable, water-insoluble organic or inorganic material capable, under the most favorable conditions, of absorbing at least about ten times its weight and, more desirably, at least about thirty times its weight in an aqueous solution containing about 0.9 weight percent sodium chloride.

These terms can be defined with additional language in the remaining portions of the specification.

### Detailed Description

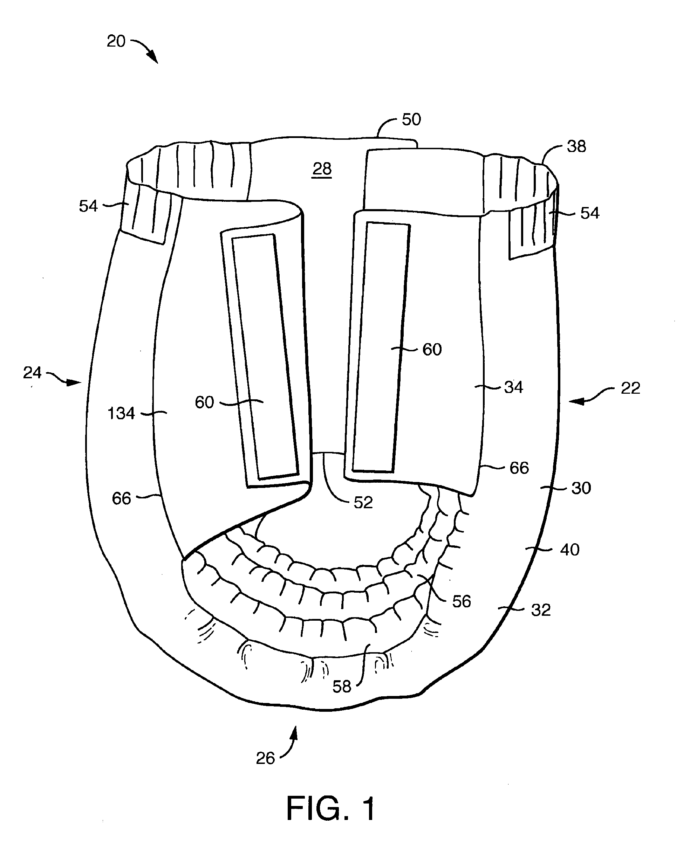

Referring now to the drawings and in particular to Figs. 1-3, an absorbent article of the present disclosure is representatively illustrated in the form of children's toilet training pants and is indicated in its entirety by the reference numeral 20. The pants 20 include a physical sensation member 70 that is adapted to create a distinct physical sensation to the wearer upon urination, which can enhance a wearer's ability to recognize when urination is occurring. The pants 20 can be disposable, which refers to articles that are intended to be discarded after a limited period of use instead of being laundered or otherwise conditioned for reuse. It should also be understood that the present disclosure is suitable for use with various other absorbent articles intended for personal wear, including but not limited to diapers, feminine hygiene products, incontinence products, medical garments, surgical pads and bandages, other personal care or health care garments, and the like without departing from the scope of the present disclosure.

By way of illustration only, various materials and methods for constructing training pants such as the pants 20 of the various aspects of the present disclosure are disclosed in PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al., and U.S. Patent 6,645,190 issued November 11, 2003 to Olson et al.. In addition, absorbent articles including a temperature change member are described in U.S. Patent No. 5,681,298 to Brunner et al..

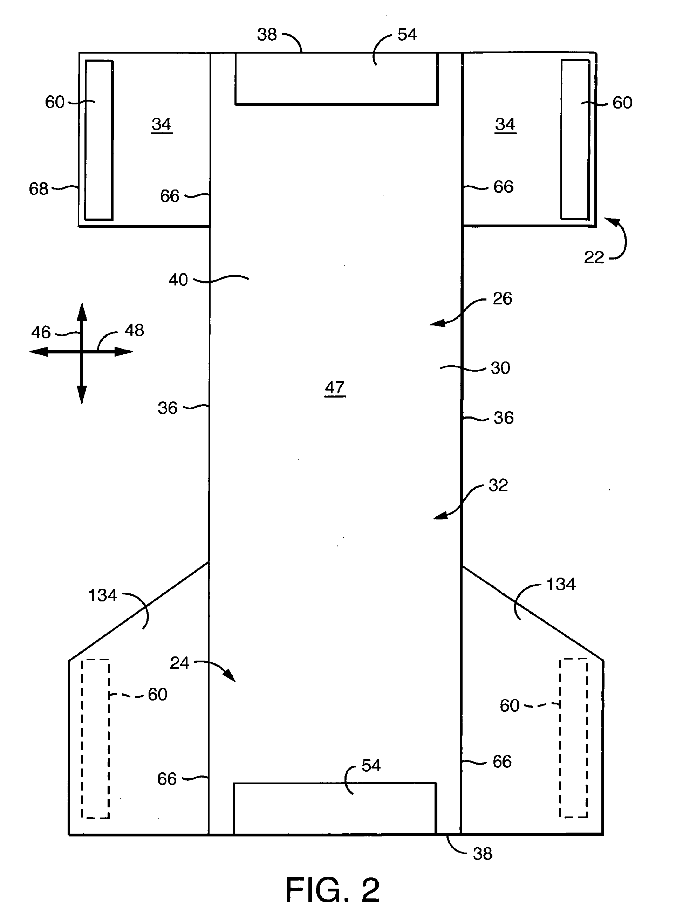

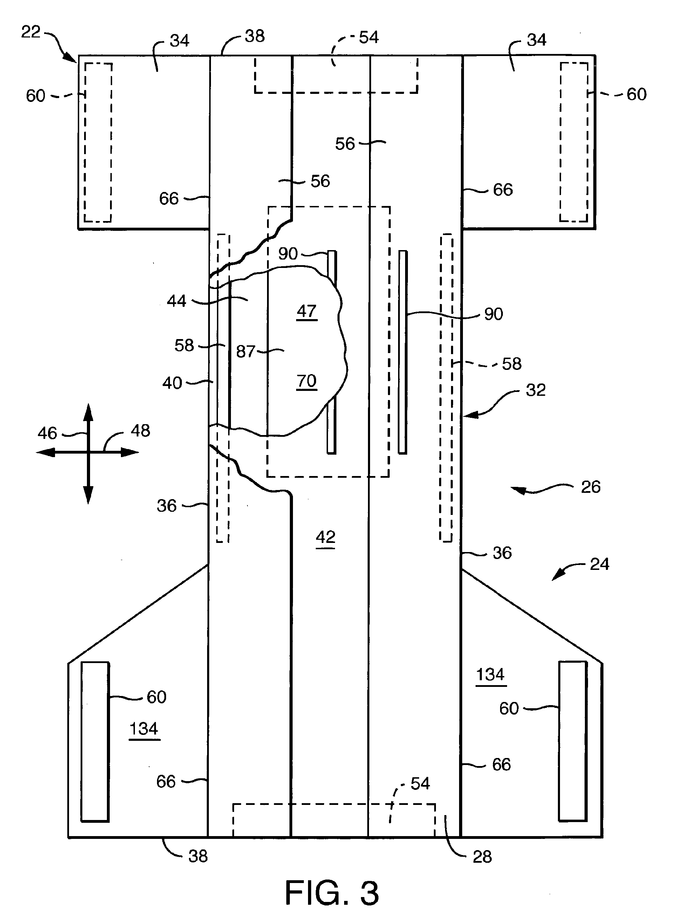

The pair of training pants 20 is illustrated in Fig. 1 in a partially fastened condition. The pants 20 define a longitudinal direction 46 and a lateral direction 48 perpendicular to the longitudinal direction as shown in Figs. 2 and 3. The pants 20 further define a pair of longitudinal end regions, otherwise referred to herein as a front waist region 22 and a back waist region 24, and a center region, otherwise referred to herein as a crotch region 26, extending longitudinally between and interconnecting the front and back waist regions 22, 24. The front and back waist regions 22, 24 includes those portions of the pants 20, which when worn, wholly or partially cover or encircle the waist or mid-lower torso of the wearer. The crotch region 26 generally is that portion of the pants 20 which, when worn, is positioned between the legs of the wearer and covers the lower torso and crotch of the wearer. The pants 20 also define an inner surface 28 adapted in use to be disposed toward the wearer, and an outer surface 30 opposite the inner surface 28. With additional reference to Figs. 2 and 3, the pair of training pants 20 has a pair of laterally opposite side edges 36 and a pair of longitudinally opposite waist edges 38 (broadly, longitudinal ends).

The illustrated pants 20 can include an absorbent assembly, generally indicated at 32. For example, in the aspect of Figs. 2 and 3, the pants 20 include a generally rectangular central absorbent assembly 32 and side panels 34, 134 formed separately from and secured to the central absorbent assembly. The side panels 34, 134 can be bonded along seams 66 to the absorbent assembly 32 in the respective front and back waist regions 22 and 24 of the pants 20. More particularly, the front side panels 34 can be permanently bonded to and extend laterally outward from the absorbent assembly 32 at the front waist region 22, and the back side panels 134 can be permanently bonded to and extend laterally from the absorbent assembly 32 at the back waist region 24. The side panels 34 and 134 can be bonded to the absorbent assembly 32 using attachment means known to those skilled in the art such as adhesive, thermal or ultrasonic bonding.

The front and back side panels 34 and 134, upon wearing of the pants 20, thus include the portions of the pants 20 that are positioned on the hips of the wearer. The front and back side panels 34 and 134 can be permanently bonded together to form the three-dimensional configuration of the pants 20, or be releasably connected with one another such as by a fastening system 60 of the illustrated aspects.

Suitable materials and structures are described further in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 issued September 10, 1991 to Vogt et al.. As is known in the art, the side panels 34, 134 can include elastic material or stretchable but inelastic materials.

The absorbent assembly 32 is illustrated in Figs. 2 and 3 as having a rectangular shape. However, it is contemplated that the absorbent assembly 32 can have other shapes (e.g., hourglass, T-shaped, I-shaped, and the like) without departing from the scope of this disclosure. It is also understood that the side panels 34, 134 can alternatively be formed integrally with the absorbent assembly 32 without departing from the scope of this disclosure. In such a configuration, the side panels 34 and 134 and the absorbent assembly would include at least some common materials, such as the bodyside liner 42, outercover 40, other materials and/or combinations thereof.

The absorbent assembly 32 includes an outercover 40 and a bodyside liner 42 (Figs. 2 and 3) in a superposed relation therewith. The liner 42 can be suitably joined to the outercover 40 along at least a portion of the longitudinal ends of the pants 20. The liner 42 can be suitably adapted, i.e., positioned relative to the other components of the pants 20, to contact the wearer's skin during wear of the pants. The absorbent assembly 32 also includes an absorbent body 44 (Fig. 3) disposed between the outercover 40 and the bodyside liner 42 for absorbing liquid body exudates. The liner 42 can be suitably joined to the outercover 40 along at least a portion of the longitudinal ends of the pants 20. The bodyside liner 42 and the outercover 40 can, for example, be attached to each other by adhesive, ultrasonic bonding, thermal bonding or by other suitable attachment techniques known in the art. Moreover, at least a portion of the absorbent body 44 can optionally be attached to the bodyside liner 42 and/or the outercover 40 utilizing the methods described above.

As mentioned above, the front and back side panels 34 and 134 can be releasably connected with one another such as by the fastening system 60 of the illustrated aspect. With the pants 20 in the fastened position as partially illustrated in Fig.1, the front and back waist regions are connected together to define the three-dimensional pants configuration having a waist opening 50 and a pair of leg openings 52. The waist edges 38 (e.g., longitudinal ends) of the pants 20 are configured to encircle the waist of the wearer to define the waist opening 50 (Fig. 1) of the pants.

The fastening system 60 can include any refastenable fasteners suitable for absorbent articles, such as adhesive fasteners, cohesive fasteners, mechanical fasteners, or the like. In one aspect of the disclosure, the fastening system includes mechanical fastening elements for improved performance. Suitable mechanical fastening elements can be provided by interlocking geometric-shaped materials, such as hooks, loops, bulbs, mushrooms, arrowheads, balls on stems, male and female mating components, buckles, snaps, or the like.

The pants 20 can further include a pair of containment flaps 56 for inhibiting the lateral flow of body exudates. As illustrated in Fig. 1, the containment flaps 56 can be operatively attached to the pants 20 in any suitable manner as is well known in the art. In particular, suitable constructions and arrangements for the containment flaps 56 are generally well known to those skilled in the art.

To further enhance containment and/or absorption of body exudates, the pants 20 can include waist elastic members 54 in the front and/or back waist regions 22 and 24 of the pants 20. Likewise, the pants 20 can include leg elastic members 58, as are known to those skilled in the art. The waist elastic members 54 and the leg elastic members 58 can be formed of any suitable elastic material that is well known to those skilled in the art. For example, suitable elastic materials include sheets, strands or ribbons of natural rubber, synthetic rubber, or thermoplastic elastomeric polymers. In one aspect of the disclosure, the waist elastics and/or the leg elastics can include a plurality of dry-spun coalesced multi-filament spandex elastomeric threads.

The outercover 40 can suitably include a material that is substantially liquid impermeable. The outercover 40 can be provided by a single layer of liquid impermeable material, or more suitably include a multi-layered laminate structure in which at least one of the layers is liquid impermeable. In particular aspects, the outer layer can suitably provide a relatively cloth-like texture to the wearer. A suitable liquid impermeable film for use as a liquid impermeable inner layer, or a single layer liquid impermeable outercover 40 is a 0.025 millimeter (1.0 mil) polyethylene film. Alternatively, the outercover 40 can include a woven or non-woven fibrous web layer that has been totally or partially constructed or treated to impart the desired levels of liquid impermeability to selected regions that are adjacent or proximate the absorbent body.

The outercover 40 can also be stretchable, and in some aspects it can be elastomeric. For example, such an outercover material can include a 0.3 osy polypropylene spunbond that is necked 60 percent in the lateral direction 40 and creped 60 percent in the longitudinal direction 48, laminated with 3 grams per square meter (gsm) Bostik-Findley H2525A styrene-isoprene-styrene based adhesive to 8 gsm PEBAX 2533 film with 20 percent TiO₂ concentrate.

The bodyside liner 42 is suitably compliant, soft-feeling, and nonirritating to the wearer's skin. The bodyside liner 42 is also sufficiently liquid permeable to permit liquid body exudates to readily penetrate through its thickness to the absorbent body 44. A suitable liquid permeable bodyside liner 42 is a nonwoven polyethylene/polypropylene bicomponent web having a basis weight of about 27 gsm; the web can be spunbonded or a bonded carded web. Optionally, the bodyside liner 42 can be treated with a surfactant to increase the wettability of the liner material.

Alternatively, the bodyside liner 42 can also be stretchable, and in some aspects it can be elastomeric. For instance, the liner 42 can be a non-woven, spunbond polypropylene fabric composed of about 2 to 3 denier fibers formed into a web having a basis weight of about 12 gsm that is necked approximately 60 percent. Strands of about 9 gsm KRATON G2760 elastomer material placed eight strands per inch (2.54 cm) can be adhered to the necked spunbond material to impart elasticity to the spunbond fabric. The fabric can be surface treated with an operative amount of surfactant, such as about 0.6 percent AHCOVEL Base N62 surfactant, available from ICI Americas, a business having offices in Wilmington, Del., U.S.A. Other suitable materials can be extensible biaxially stretchable materials, such as a neck stretched/creped spunbond. Reference is made to U.S. Patent No. 6,552,245, issued April 22, 2003, to Roessler et al..

An absorbent body 44 can be disposed on the outercover 40, for example, between the outercover 40 and the bodyside liner 42. The outercover 40 and the bodyside liner 42 can be joined together by any suitable means such as adhesives, ultrasonic bonds, thermal bonds, or the like. The absorbent body 44 can be in a variety of shapes and configurations as are known in the art, such as rectangular, hourglass shaped, I-shaped, and the like. Further, at least a portion of the absorbent body 44 can optionally be attached to the bodyside liner 42 and/or the outercover 40 utilizing the methods described above.

The absorbent body 44 is suitably compressible, conformable and capable of absorbing and retaining liquid body exudates released by the wearer. For example, the absorbent assembly can include a matrix of absorbent fibers, and more suitably cellulosic fluff, such as wood pulp fluff, and superabsorbent particles. As an alternative to wood pulp fluff, synthetic fibers, polymeric fibers, meltblown fibers, short cut homofil bicomponent synthetic fibers, or other natural fibers can be used. Suitable superabsorbent materials can be selected from natural, synthetic, and modified natural polymers and materials. The superabsorbent materials can be inorganic materials, such as silica gels, or organic compounds, such as crosslinked polymers, for example, sodium neutralized polyacrylic acid.

The absorbent body 44 can have a density within the range of about 0.10 to about 0.5 grams per cubic centimeter and can be wrapped or encompassed by a suitable tissue or nonwoven wrap for maintaining the integrity and/or the shape of the absorbent assembly.

In one aspect, the absorbent body 44 can be stretchable so as not to inhibit the stretchability of other components to which the absorbent body can be adhered, such as the outercover 40 and/or the bodyside liner 42.

In some aspects, a surge management layer (not shown) can be included in the pants 20. The surge management layer can be positioned in the pants 20 in a variety of locations as is known in the art. For example, the surge management layer can be proximate the absorbent body 44, for example between the absorbent body 44 and the bodyside liner 42, and attached to one or more components of the pants 20 by methods known in the art, such as by adhesive, ultrasonic or thermal bonding. In addition, the surge management layer can be positioned in the pants 20 relative to the physical sensation member 70 in a variety of ways. For instance, the surge management layer can be disposed toward the liner 42 relative to the physical sensation member 70, or the surge management layer can be disposed toward the absorbent body 44 relative to the physical sensation member 70.

A surge management layer helps to decelerate and diffuse surges or gushes of liquid that can be rapidly introduced into the absorbent body 44. Desirably, the surge management layer can rapidly accept and temporarily hold the liquid prior to releasing the liquid into the storage or retention portions of the absorbent body 44.

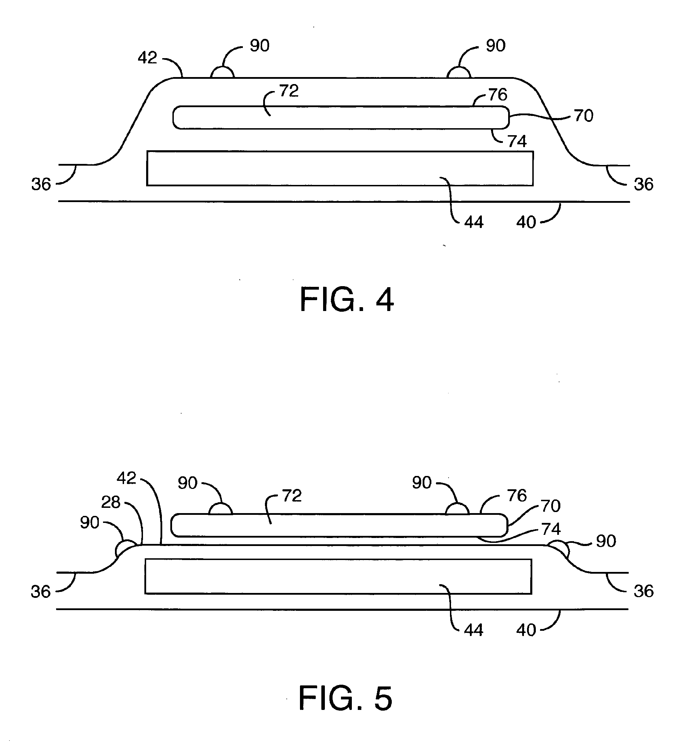

As mentioned above, in the various aspects of the absorbent article of the present disclosure, the pants 20 can also include a physical sensation member 70 (Figs. 2 and 3). The physical sensation member 70 can include a physical sensation composite 72 and optionally a first carrier layer 74 in a superposed relationship with physical sensation composite 72 (Figs. 4 and 5). The physical sensation member 70 can also optionally include a second carrier layer 76 where the first carrier layer 74 and the second carrier layer 76 sandwich the physical sensation composite 72 (Figs. 4 and 5). A more detailed description of the various options with respect to such a physical sensation member 70 can be found on U.S. Patent Application Serial No. 11/521,801.

The physical sensation member 70 is disposed within the pants 20 so that, upon urination, liquid makes contact with the physical sensation composite 72. The physical sensation member 70 is disposed with the absorbent body 44, intermediate the outercover 40 and liner 42. In particular, the physical sensation member 70 can be attached to the absorbent body 44 and disposed toward the interior surface of the pants 20. Alternatively, the physical sensation member 70 can be attached to the liner 42 adjacent the absorbent body 44. In another aspect, the physical sensation member 70 can be disposed within a gap between portions of the absorbent body 44 and attached, for example, to the outercover 40. In still another aspect, the physical sensation member 70 can be attached to the inner surface 28 of the liner 42.

As can be readily appreciated, the physical sensation member 70 can be of various shapes and sizes. For example, the physical sensation member 70 can be rectangular and can have a width in the lateral direction 48 of from 2.5 cm to 10 cm and a length in the longitudinal direction 46 of from 2.5 cm to 25 cm. In one aspect the physical sensation member 70 can measure about 8 cm by about 10 cm. Alternatively, the physical sensation member 70 can be oval in shape, circular, triangular, or the like. In yet another alternative, the physical sensation member 70 can generally be provided in strips that extend in the lateral 48 or longitudinal direction 46 and that can be separated by a gap of about 2.5 cm.

The physical sensation composite 72 can include a matrix of fibers and physical sensation material intermixed within the matrix of fibers. The matrix of fibers can be substantially continuous or discrete and discontinuous. In addition, the matrix of fibers of the physical sensation composite 72 can be provided by a variety of different fibers as are known in the art. For example, the matrix of fibers can include adhesive fibers, absorbent fibers, binders (including binder fibers), polymer fibers, and the like or combinations thereof. As such, the physical sensation material can be suitably entrapped within the matrix to limit material shake-out or loss during manufacture and/or wear of the pants 20.

In particular, in aspects where the matrix of fibers includes adhesive fibers, the fibers can be provided by a hot-melt adhesive. Such an adhesive generally comprises one or more polymers to provide cohesive strength, a resin or analogous material, perhaps waxes, plasticizers or other materials to modify viscosity, and/or other additives including, but not limited to, antioxidants or other stabilizers.

As an example, a suitable hot-melt adhesive can contain from about 15 to about 50 weight percent cohesive strength polymer or polymers; from about 30 to about 65 weight percent resin or other tackifier or tackifiers; from more than zero to about 30 weight percent plasticizer or other viscosity modifier; and optionally less than about 1 weight percent stabilizer or other additive. It should be understood that other hot-melt adhesive formulations comprising different weight percentages of these components are possible.

Examples of a suitable adhesive for use in providing the matrix of fibers are hot-melt adhesives available from H.B. Fuller Adhesives of Saint Paul, Minnesota under the designation HL8151-XZP. In particular, this adhesive is a hydrophilic adhesive that promotes the rapid wettability of the physical sensation member 70 resulting in faster physical sensation. Alternatively, it is contemplated that the adhesive can be a hydrophobic adhesive without departing from the scope of the present disclosure. It is also contemplated that alternative adhesives can be used without departing from the scope of this disclosure.

The matrix of fibers can be produced by adhesive fibers in a variety of adhesive application processes as are known in the art. For example, adhesive can be meltblown onto a foraminous surface, such as a screen, or onto a substrate such as the first carrier layer 74 that can be placed onto the foraminous surface. In particular, a suitable adhesive applicator system can be used to apply the adhesive in the form of discrete fibers or filaments. For instance, the resulting matrix of fibers can have an adhesive fiber diameter in the range of about 5 microns to about 200 microns, and more suitably in the range of about 7 microns to about 50 microns.

In one aspect, adhesive fibers can have a basis weight in the range of about 1 to about 150 grams per square meter (gsm), and more suitably in the range of about 50 to about 100 gsm to form the matrix of fibers. Similarly, the physical sensation material can have a basis weight in the range of about 500 to about 2000 grams per square meter (gsm), and more suitably in the range of about 1000 to about 1500 gsm to form the matrix of fibers. In another aspect, the amount of adhesive used in forming the physical sensation composite is suitably less than or equal to about 20 percent by weight of the amount of physical sensation material used in forming the physical sensation composite 72, more suitably less than or equal to about 10 percent by weight of the amount of physical sensation material used in forming the physical sensation composite 72, and still more suitably in the range of about 5 to about 10 percent by weight. In a particular aspect, the physical sensation composite can be 95 gsm adhesive and 1400 gsm physical sensation material 80.

The physical sensation material can be intermixed with the adhesive fibers providing the matrix of fibers by being fed into and entrained in a stream of adhesive to form a blended mixture of adhesive fibers and physical sensation material 80 that can be applied to a substrate, such as the first carrier layer 74. Optionally, the second carrier layer 76 can overlay the physical sensation composite 72 and be secured thereto by the adhesive in the matrix of fibers.

In such an aspect, the physical sensation member 70 can optionally be subjected to further processing. For example, as will be described in greater detail below, a compressive force can be applied to the physical sensation member 70 to facilitate strengthening of the bonds between the matrix of fibers and the physical sensation composite 72 as well as between the matrix of fibers and any carrier layers that can optionally be present. The compression can also reduce the thickness, or caliper of the physical sensation member 70 such that it can be more discreetly included in the pants 20.

Alternatively, the matrix of fibers of the physical sensation composite 72 can include absorbent fibers. Suitable absorbent fibers can include natural absorbent fibers such as cellulosic fibers (i.e., wood pulp fibers) or cotton fibers, synthetic absorbent fibers such as rayon or cellulose acetate or combinations thereof. In particular, the absorbent fibers can be mixed bleached southern softwood and hardwood Kraft pulp, or any other suitable fibers.

Optionally, in such an aspect, the matrix of fibers can further include binder material. For example, the binder material can suitably be a thermoplastic binder material. Such binder materials can soften when exposed to heat and can substantially returns to their original condition when cooled to room temperature. Such thermoplastic binder materials, when in the softened state, constrain or entrap the fibers and other materials proximate the binder to stabilize the physical sensation composite 72. Binder materials can be provided in powder or fiber form. Examples of suitable binder materials for use with the present disclosure can be those having low melting temperatures such as polyethylene glycol (PEG) or paraffin wax.

In such an aspect, the matrix of fibers can be provided by absorbent fibers by forming the matrix on a forming surface of a conventional air-forming device. Such air-forming devices are well known to those skilled in the art for use in forming fibrous webs. In such devices, fibrous material is introduced and can be mixed with other material such as the physical sensation material prior to collecting on the forming surface. A pneumatic flow mechanism, such as a vacuum suction system, draws the air-entrained fiber stream within the air-forming device toward the forming surface so that air passes through the foraminous surface while the fibers and other air-entrained material collect on the forming surface.

Thus, a matrix of absorbent fibers and physical sensation material intermixed with the matrix can be collected on the forming surface to form the physical sensation composite 72. Optionally, an air permeable substrate can be laid upon the forming surface to collect the physical sensation composite 72 and provide a first carrier layer 74. Further a second carrier layer 76 can be placed upon the physical sensation composite following the formation of the physical sensation composite in the air-forming device to increase the integrity of the physical sensation member 70.

The physical sensation member 70 (with or without the first and second carrier layers 74 and 76) can be further processed, such as by passing the physical sensation member through a nip defined by opposed rolls in order to compress it down to a uniform thickness. Following compression in this manner, the physical sensation member 70 can define a density of between .20 grams per cm³ to .55 grams per cm³, particularly a density of between .25 grams per cm³ to .45 grams per cm³ and still more particularly, a density of .35 grams per cm³. Alternatively, other densification methods can be utilized as are well known to those skilled in the art. Densities within these ranges are believed to provide a flexible physical sensation member 70 that retains the physical sensation material within the matrix of fibers and that has desirable integrity. Moreover, such densities are not so high as to crush or otherwise impair the physical sensation material thereby reducing its efficacy. In addition, in aspects where the physical sensation composite 72 includes binder material, the physical sensation member 70 can optionally be subjected to heat activation such as by heat calendar rolls or a through air heating device.

The physical sensation composite 72 including absorbent fibers as described above can be from 5 to 50 percent by weight absorbent fibers, and from 50 to 95 percent by weight physical sensation material. Alternatively, the physical sensation composite 72 can be from 20 to 40 percent by weight absorbent fibers, and from 60 to 80 percent by weight physical sensation material 80. Optionally, the composite 72 can include between 1 and 15 percent by weight binder fibers. In a particular aspect, the physical sensation composite 72 can be 70 percent by weight physical sensation material 80 and 30 percent by weight absorbent fibers and define a density of .35 grams per cm³.

In yet another alternative the matrix of fibers can be provided by a coform composite including polymer fibers and absorbent fibers. Coform materials and coforming processes are known in the art.

In a particular aspect, the matrix of fibers can be provided by a coform composite that can be a blend of meltblown polymer fibers and cellulosic fibers. Various suitable materials can be used to provide the meltblown fibers such as a polyolefin material. Alternatively, the polymer fibers can be stretch polymer fibers, such as those provided by a copolymer resin. Other suitable polymeric materials or combinations thereof can alternatively be utilized as are known in the art. Further, various absorbent cellulose fibers can be utilized.

The polymer fibers and the meltblown fibers can be coformed to provide the matrix of fibers by providing a stream of absorbent fibers and a stream of extruded molten polymeric fibers. Further, to provide the physical sensation composite 72, a stream of physical sensation material can also be provided. These streams can be merged into a single stream and collected on a forming surface such as a forming belt or forming drum to form the physical sensation composite 72 of the physical sensation member 70. Optionally, a forming layer, such as first carrier layer 74, can be placed on the forming surface and used to collect the materials included in the physical sensation composite 72.

The stream of absorbent fibers can be provided by feeding a pulp sheet into a fiberizer, hammermill, or similar device as are known in the art. The stream of polymer fibers can be provided by meltblowing a copolymer resin or other polymer. In particular, the melt temperature for a copolymer resin such as VM2210 can be from 450 degrees F (232 degrees C) to 480 degrees F (248 degrees C) to improve the entrainment of the physical sensation material in the matrix. As mentioned above, suitable techniques for producing nonwoven fibrous webs, which include meltblown fibers, are described in the previously referenced U.S. Patent Nos. 4,100,324 and 5,350,624. The meltblowing techniques can be readily adjusted in accordance with conventional know-how to provide turbulent flows that can operatively intermix the fibers and the physical sensation material 80. For example, the primary air pressure can be set at 5 psi and the meltblown nozzles can be 0.020 inch spinneret hole nozzles. The techniques can also be readily adjusted in accordance with conventional knowledge to provide the desired weight percentages of the various materials in the physical sensation composite 72.

The stream of physical sensation material can be pneumatically provided or gravity fed. A suitable method and apparatus for delivering material in an airstream is described in U.S. patent no. 6,461,086, issued October 8, 2002 to Milanowski et al. The coform material can also include other materials, such as superabsorbent materials.

In one aspect, the physical sensation composite 72 provided by a coform composite as described above can be from 5 to 15 percent by weight meltblown polymer fibers, 10 to 50 percent by weight absorbent fibers and 40 to 80 percent by weight physical sensation material. In a particular aspect, the physical sensation composite 72 can be 8 percent by weight meltblown polymer fibers, 14 percent by weight absorbent fibers, and 78 percent by weight physical sensation material, with a basis weight of 1340 gsm.

As mentioned above, the physical sensation member 70 can optionally include a first carrier layer 74 (Figs. 4 and 5) in superposed relationship with the physical sensation composite 72. Further, the physical sensation member 70 can optionally include a first carrier layer 74 and a second carrier layer 76 (Figs. 4 and 5) where the first carrier layer and the second carrier layer 76 sandwich the physical sensation composite 72. The first and second carrier layers 74 and 76 can be provided by separate webs of material, or alternatively can be provided by a single web of material that is folded in half about the physical sensation composite 72.

In certain aspects, the carrier layers 74 and 76 can be liquid permeable or liquid impermeable. For instance, one carrier layer, such as the first carrier layer 74 can be liquid impermeable and the other carrier layer, (i.e., the second carrier layer 76 can be liquid permeable. As such, liquid insults can pass through the second carrier layer 76 to activate the physical sensation material, and the first carrier layer 74 can slow the flow of the liquid insult from leaving the physical sensation member 70 thus maximizing the physical sensation that can be felt by the wearer. Alternatively, the first carrier layer 74 can be liquid permeable, and in aspects with a second carrier layer 76, both carrier layers 74 and 76 can be liquid permeable. Such carrier layers 74 and 76 can further improve the integrity of the physical sensation member 70 for improved processability and can also aid in retaining the physical sensation material within the member 70.

Suitable liquid permeable materials for the carrier layers 74 and 76 include tissue layers, nonwoven layers, or combinations thereof. In particular, materials described as suitable for use as the bodyside liner 42 can also be suitable for a liquid permeable carrier layer 74 and 76. Accordingly, a liquid permeable carrier layer 74 and 76 can also be stretchable. Likewise, materials described as suitable for use as the outercover 40 can be suitable for use as a liquid impermeable carrier layer 74 and 76. Accordingly, a liquid impermeable carrier layer 74 and 76 can also be stretchable.

The physical sensation provided to the wearer can be enhanced by increasing the contact between the wearer and the physical sensation member and the liner 42. To maximize the effectiveness of the physical sensation reaction as a training aid, the physical sensation member 70 should be in close contact with the wearer's skin as the wearer has an insult incident. A potential position of the crotch region of pants 20 in use is in the form of a bucket, with the crotch region falling away from the wearer's body. This bucket effect works well to best utilize the absorbent body 44 in the pants 20. By using body adhesive 90 in connection with the physical sensation member 70 and the liner 42, portions or all of the physical sensation member 70 and liner 42 can cling to the wearer, holding these elements close to the wearer's skin. Such an arrangement does not adversely impact the absorbent function of the pants 20 because the rest of the absorbent system is still in bucket form.

The body adhesive 90 is applied to the inner surface 28 of the pants 20. According to the present invention the physical sensation member 70 is disposed between the absorbent body 44 and the liner 42 as illustrated in Fig. 4, the inner surface 28 is the body-facing surface of the liner 42, and the body adhesive 90 is applied thereon. In another example outside the scope of the present invention the physical sensation member 70 is disposed on the body-facing surface of the liner 42 as illustrated in Fig. 5, the inner surface 28 is the body-facing surface of the physical sensation member 70, and the body adhesive 90 is applied to the physical sensation member 70, to the liner 42 in the vicinity of the physical sensation member 70, or in both locations.

Body adhesives have been used in health care products such as on wound dressings and consumer products such as bandages for many years. For the present disclosure, the body adhesive 90 should exhibit good stay-in-place performance and easy removal performance. Suitable body adhesives include low peel adhesives such as PSA-7-9700 silicone skin adhesive and Bio-PSA 7-4560 silicone hot-melt adhesive available from Dow Corning of Midland, MI, and OLEEVA-brand silicone skin adhesive material available from Bio Med Sciences, Inc. of Allentown, PA. For situations in which high breathability is preferred, an acrylic-based adhesive can be more suitable. Suitable acrylic-based adhesives include an acrylic hot melt adhesive such as DERMA-TAK 546B-brand acrylic hot melt adhesive available from National Starch and Chemical Company, Bridewater, NJ.

The body adhesive 90 can be applied to the physical sensation member 70 and/or the liner 42 in a continuous pattern such as through slot coating or in a discontinuous pattern such as beads, ribbons, or meltblown fibers to obtain the optimum performance of stretch and strength. The body adhesive 90 can be applied at a rate of 3 grams per square meter (gsm) to 100 gsm or higher. The tack or peel strength of the body adhesive 90 can be measured. Typical values for the application of the present disclosure are in the range of 10-100 grams per inch off of human skin.

As noted previously, the physical sensation member 70 is positioned and adapted to create a distinct physical sensation upon the pants 20 being insulted. The physical sensation can be a temperature change such as cooling or heating, can be a pressure change such as from an expandable element, or can be a foaming, fizzing, bubbling, or other physical sensation.

A physical sensation in the form of a temperature change can result from a temperature change material that is in the form of particles captured between the pair of carrier layers. The carrier layers 74, 76 house and limit movement of the temperature change material. In this aspect, the physical sensation material is a temperature change material.

The temperature change material of the various aspects of the present disclosure can include a substance that provides a temperature change when placed near the wearer and contacted with urine. The temperature change can be either an absorption or release of heat that is noticeable to the wearer. Absorption of heat by the temperature change material will provide the wearer with a cool sensation, while a release of heat by the substance will provide the wearer with a warm sensation. Reference is made to U.S. Patent Application Serial No. 10/462,166, in the name of Olson, et al., for additional information regarding the mechanism by which the temperature change sensation is accomplished. Suitably, the temperature change material can be provided in particulate form for ease of processing in the described aspects.

The physical sensation material can be homogeneously intermixed within the matrix of fibers. Alternatively, the physical sensation material can define a distribution gradient within the thickness of the physical sensation composite 72. For example, the physical sensation material can be intermixed within the matrix of fibers in greater amounts toward the interior surface 28 of the pants. Alternatively, the physical sensation material can be intermixed within the matrix of fibers in greater amounts toward the exterior surface 30 of the pants.

The temperature change material is responsive to contact with an aqueous solution such as urine to either absorb or release heat. The mechanism by which this is accomplished is the dissolution of the substance in the aqueous solution, the swelling of the substance in the aqueous solution, or the reaction of the substance in the aqueous solution. In particular aspects, the temperature change material is a particle that has a substantial energy difference between a dissolved state and a crystalline state, so that energy in the form of heat is absorbed or released to the environment upon contact with urine. In other aspects, the temperature change material releases or absorbs energy during swelling or reacting of the substance in an aqueous solution.

While a wide variety of substances can result in a temperature change when contacted with an aqueous solution, the selection of a particular temperature change material, the determination of the amount to be used and the location of the substance should be based in part on the desired temperature change. Specifically, the physical sensation member 70 can suitably provide the training pants 10 with a temperature change (i.e., cooler or warmer) when wet of at least about 5 degrees C, more suitably about 10 degrees C, still more suitably about 15 degrees C. Alternatively, the physical sensation member 70 can provide the pant 20 with a surface temperature change when wet of from 5 degrees C to 15 degrees C. Surface temperature changes within this range are believed to be identifiable to some extent by children of toilet training age. More suitably the physical sensation member 70 can provide the training pant 10 with a surface temperature change when wet of from 5 degrees C to 10 degrees C.

To achieve this result, the temperature change material, the amount used, and the location of the substance should be selected so that the possible total energy change is from about 6 to about 30 calories per square centimeter (cal/cm²), which can represent either a possible total energy release of from about 6 to about 30 cal/cm² or a possible total energy absorption of from about 6 to about 30 cal/cm². More desirably, the temperature change material, the amount used, and the location of the substance should be selected so that the possible total energy change is from about 12 to about 24 cal/cm², and more particularly about 18 cal/cm².

Thus, in a particular aspect, where the temperature change material is endothermic, a drop in the temperature of the product when insulted can be from about 37 degrees C to about 25 degrees C, and further to about 22 degrees C for improved effectiveness, particularly with a preoccupied wearer (i.e., a playing child). The temperature change can suitably last for at least 10 minutes, and more suitably for approximately 15 minutes.

By way of example, urea particles can be selected to provide a cooling sensation, because urea particles absorb heat when dissolved in an aqueous solution. Urea has a heat of solution of approximately -60 calories per gram (cal/g). A desirable add-on amount for the urea particles would be a basis weight of about 0.3 grams per square centimeter (g/cm²). The selection of urea particles at this basis weight results in a possible total energy change of 60 cal/g×0.3 g/cm² which equals 18 cal/cm².

Temperature change materials that absorb or release heat on contact with an aqueous solution desirably have a heat of solution, hydration, or reaction greater than about 40 cal/g or less than about -40 cal/g. The heat of solution, hydration, or reaction is suitably within the range of from about 40 to about 90 cal/g or from about -40 to about -90 cal/g, and more particularly from about 50 to about 70 cal/g or from about -50 to about -70 cal/g, such as urea at -60 cal/g. Suitable basis weights for such temperature change materials range from about 0.1 to about 0.5 g/cm², and more particularly from about 0.2 to about 0.4 g/cm².

As referenced above, temperature change materials suitable for use in the pants 20 include those that dissolve in an aqueous solution. The solubility of such temperature change materials is desirably from about 0.1 to about 3 grams of water (H₂O) per gram of material (g/g), and more particularly from about 0.1 to about 2 g/g for improved performance.

Suitable temperature change materials that absorb heat during dissolution can include salt hydrates, such as sodium acetate (H₂O), sodium carbonate (10H₂O), sodium sulfate (10H₂O), sodium thiosulfate (5H₂O), and sodium phosphate (10H₂O); anhydrous salts, such as ammonium nitrate, potassium nitrate, ammonium chloride, potassium chloride, and sodium nitrate; organic compounds, such as urea, xylitol, and other sugars; or the like. Temperature change materials that release heat during dissolution can include aluminum chloride, aluminum sulfate, potassium aluminum sulfate, or the like. The temperature change material can also include those substances that absorb or release heat during swelling. By way of illustration, one suitable temperature change particle that releases heat during swelling is a lightly cross-linked partially neutralized polyacrylic acid.

The temperature change material can also include those substances that absorb or release heat during swelling. By way of illustration, one suitable temperature change material that releases heat during swelling is a lightly cross-linked partially neutralized polyacrylic acid. Other temperature change material that releases heat during dissolution includes aluminum chloride, aluminum sulfate, potassium aluminum sulfate, or the like.

Alternatively, the temperature change material can include those substances that absorb or release heat upon reaction with an aqueous solution. Examples include ortho esters or ketals such as menthone ketals that result from reacting menthone with alcohols containing 1 to 8 carbons or polyols containing 2 to 8 carbons, and all structural and optical isomers thereof. Particular menthone ketals that can be suitable include menthone-glycerol ketal and menthone-propylene glycol ketal. Particular ketals are disclosed in U.S. Pat. No. 5,348,750 issued Sep. 20, 1994, to Greenberg; and in U.S. Pat. No. 5,266,592 issued Nov. 30, 1993, to Grub et al..

The temperature change material is desirably although not necessarily in the form of particles sandwiched between the first and second carrier layers 74, 76. The first carrier layer 74 can, for example, include a porous film or fibrous layer. The fibrous layer can include a fibrous tissue, a woven or nonwoven fabric, a cellulosic fibrous web, or the like. In one aspect, for example, the first carrier layer 74 can include a cellulosic tissue composed of a conventional forming tissue having a basis weight of about 16.6 gsm and manufactured by a continuous wet press (CWP) process from a furnish composed of 100% LL-19 Northern Softwood Kraft (NSWK) fiber. The forming tissue has a Frazier Porosity of about 50-350 cfm/ft² (cubic-feet-per-minute per square foot).

The second carrier layer 76 can, for example, include a liquid-permeable web material, such as a liquid-permeable film, tissue, fabric, or the like. The fabric can be woven or nonwoven, and can be composed of a hydrophilic material or composed of a hydrophobic material that has been suitably treated to render it sufficiently hydrophilic. In one aspect, the second carrier layer 76 includes a conventional barrier tissue having a basis weight of about 21.2 gsm and manufactured by a CWP machine process from a furnish composed of 50%/50% Hinton EF (Softwood) and LL-16 Northern Hardwood Kraft (NHWK) fiber. The barrier tissue can have a Frazier Porosity of about 80-120 cfm/ft².

The physical sensation member 70 desirably provides a surface temperature change when wet of from about 5 to about 25 degrees Fahrenheit (°F) (2. 8°-13.8°C) at the surface of the liner 42. Surface temperature changes within this range are believed to be identifiable to most absorbent article wearers. More desirably, the training pant 20 provides a surface temperature change when wet of from about 10° to about 20°F (5.5°-11.1°C), and particularly about 15°F (8.3°C) for improved performance. Also, the cool or warm sensation produced by the physical sensation member 70 should last from about 1 to about 120 seconds, and particularly from about 10 to about 60 seconds, such as about 30 seconds.

In an alternative aspect of the disclosure, the temperature change particles are desirably accumulated in a plurality of pockets. Without wishing to be bound by any particular theory, positioning the temperature change particles in discrete pockets is thought to enhance performance because the particles in the interior portions of the pockets are, for an extended period of time, damp rather than saturated. As a result, the heat taken in or released by these temperature change particles as they enter solution, swell, or react comes from the surrounding environment rather than solely from the urine or other body exudates.

Consequently, locating the temperature change particles in pockets within the physical sensation member 70 facilitates generation of a cool or a warm sensation in an efficient and cost effective manner.

Thus, as described above, the physical sensation member 70 can include a matrix of fibers 72 and the physical sensation material, such as Xylitol particles, intermixed within the matrix of fibers 72. Once wet by urination, the Xylitol dissolves, creating an endothermic response, thereby signaling to the wearer that urination has occurred.

Further, the physical sensation member 70 can suitably contain various amounts of the physical sensation material as described above. Further, it will be understood by those of skill in the art that the pants 20 of the present disclosure could include more than one physical sensation member 70.

Therefore, as can be readily appreciated, the pants 20 of the various aspects of the present disclosure provide a physical sensation member 70 that effectively signals urination to the wearer and can be readily processed in a high-speed converting process with a limited amount of physical sensation material 80 shake-out or loss.

A suitable procedure for determining the temperature change when wet of a product containing a temperature change material is described below in the temperature change test as follows. The test should be conducted in an environment having a stable temperature of 21 degrees C to 22 degrees C and a stable humidity of about 50 percent. The product to be tested is prepared by removing any elastic side panels and cutting all other elastics to permit the product to lay as flat as possible. The product is positioned in a Plexiglas cradle to simulate the configuration of the product in actual use. The center of the product is placed in the deepest portion of the cradle.

A liquid dispensing nozzle operatively connected to a liquid dispensing pump is positioned to dispense saline onto the inner surface of the product. The tip of the nozzle should be located 1 cm away from the inner surface and 10 cm forward of the center of the product, along the product's longitudinal axis. The pump is activated to dispense 90 ml of a stabilized isotonic 0.9 percent saline at a rate of 15 ml/sec. The saline is certified blood bank saline available from The Baxter Healthcare Corporation, Scientific Products Division, McGraw Park, III., and is at a temperature of 37° C.

The surface temperature of the product at the location of the physical sensation member is measured using a standard thermometer or temperature sensing thermistors connected to a digital display or recording device. The surface temperature 30 seconds after the saline is dispensed is recorded as the test temperature. A reference temperature is obtained by performing this test on a portion of the product not including the temperature change material or on a similar product without the temperature change material. The surface temperature change when wet for the product is the difference between the test temperature and the reference temperature.

In another aspect of the disclosure, the physical sensation member 70 is adapted to provide the wearer with an expanding or contraction dimensional change sensation. Dimensional change elements of this type are described in more detail in U.S. Patent No. 5,649,914 to Glaug et al.. The physical sensation member 70 includes a dimensional change member positioned within the carrier layers 74, 76. The peripheries of the carrier layers 74, 76 can be bonded directly together by adhesives, thermal bonds, ultrasonic bonds, or other suitable means.

The dimensional change member includes a material or materials that rapidly undergo a change in at least one dimension when exposed to an aqueous solution. The dimensional change is suitable either as an expansion to at least about 2 times a dry dimension or as a contraction to less than about one-half (1/2) of the dry dimension. In particular aspects, the dimensional change is either an expansion to at least about 5 times the dry dimension or a contraction to less than about one-fifth (1/5) of the dry dimension. For example, the dimensional change member has a wet height dimension that is at least about 5 times greater than its dry height dimension, and more desirably at least about 10 times greater for improved performance. The height dimension of the dimensional change member is perpendicular to the plane formed by the longitudinal and transverse axes 40, 42 of the pants 20 so that the dimensional change is noticeable to the wearer of the pants 20. The other dimensions, the width and length, of the dimensional change member can remain the same, expand, or contract when exposed to an aqueous solution.

In one particular aspect, the dimensional change member includes a compressed cellulose sponge having a dry height of about 0.9 mm and a wet height of about 9.5 mm. The height dimensions are measured with the material under a compressive load of 0.2 pounds per square inch.

The noncompressed axes of the material, that is the width and length, expand only about 7 percent from dry to wet states. Additionally, the dimensional change member is desirably generally hydrophobic so that the dimensional change member releases liquid to the pants 20.

In one aspect of the disclosure, the dimensional change member is capable of expanding to at least about 5 times its dry height in 10 seconds, and more particularly to at least about 10 times its dry height in 3 seconds for improved performance.

Suitable materials for use in the dimensional change member include expandable foams, compressed cellulose sponges, superabsorbents, or the like. Particularly desirable expandable foams include those having open, large cell, reticulated structures. Examples of such expandable foams are available from O-Cell-O, General Mills, Inc., Tonawanda, N.Y., USA, and Industrial Commercial Supply Co., Akron, Ohio, USA. The material forming the dimensional change member can be softened by mechanical means or other suitable techniques so as to be less noticeable until urination occurs. One such means that is effective with compressed cellulose sponge is to run the material through a set of meshed gears with the gap between the gears set so that the material is sufficiently scored to make it pliable.

In another aspect of the present disclosure, the dimensional change element of the physical sensation member 70 can be a urine-or-other-body-exudates-permeable inflatable container positioned between the bodyside liner 42 and the absorbent body 44. Dimensional change elements of this type are described in more detail in U.S. Patent No. 7,002,055 to Long et al.. The inflatable container includes a surfactant and a system that, upon wetting with urine or other body exudates, produces a gas, such as carbon dioxide. The gas produced upon wetting with urine or other body exudates interacts with the surfactant to produce foam that inflates the container. The inflated container pushes against the bodyside liner and causes the bodyside liner to press against the skin of the wearer to alert the wearer that the absorbent body 44 has been insulted.

The inflatable container that is positioned between the bodyside liner 42 and the absorbent body 44 includes an inflatable, liquid-permeable container. The container can be suitably formed from either woven or nonwoven substrates that are substantially liquid permeable to allow liquids, such as urine, to pass therethrough and contact the gas producing system and surfactant described herein. In one aspect, the inflatable liquid permeable container can be formed from a 20 gsm spunbond nonwoven material available from Kimberly-Clark Corporation, Neenah, Wisconsin. More particularly, a pair of opposed sheets of such material can be ultrasonically or otherwise bonded together along an edge margin about the periphery of the container so as to seal the container. The container can be either adhesively or thermally bonded to the absorbent body 44 and/or the bodyside liner 42 to stabilize the container during use. The container is sized, configured, and positioned in the pants 20 in such a manner that the container is free to swell without substantial interference from other components of the pants 20.

It should be understood that the container could be fabricated from materials other than a spunbond nonwoven so long as at least a portion of the container is sufficiently liquid permeable to permit liquid body exudates to permeate therethrough into the interior of the container for contact with the gas producing systems and surfactants described herein.

As noted above, the permeable inflatable container includes a system capable of generating a gas upon being wetted. The gas that is produced in the container upon the wetting interacts with one or more surfactants, which are discussed below, and produces foam that inflates the container and causes it to press the bodyside liner 42 against the skin of the wearer to alert the wearer that the absorbent body 44 is nearing fullness. The distortion causes the bodyside liner 42 to press against the skin of the wearer to alert the wearer to the nearing fullness of the absorbent body 44.

In one aspect, the system is capable of generating gas upon being wetted, which is located in the permeable inflatable container, includes at least one acid and at least one base. The acid and base react together upon being wetted to produce a gas that can be, for example, carbon dioxide gas. The exact gas produced by the gas producing system is not critical, so long as the gas produced is substantially non-harmful to the skin of the wearer.

In another aspect, the system capable of generating a gas upon being wetted includes a urine-or-other-body-exudates-soluble effervescent solid material produced in such a manner such that a pressurized gas is trapped within cells located in the solid material. When the solid material having pressurized gas-containing cells is contacted with urine or other body exudates, the solid material begins to dissolve and the pressurized gas is released from the cells during dissolution of the solid material. This gas can interact with the surfactant also located in the permeable inflatable container and produce foam and bubbles that inflate the container as described herein.

In this aspect, the soluble effervescent solid material can include a sugar compound such as a mono-saccharide, di-saccharide, or poly-saccharide that has been infused with a gas that is substantially non-reactive with human skin. Suitable gases for infusion into a solid material include, for example, carbon dioxide, air, nitrogen, argon, helium, other substantially inert gases, and combinations thereof. Specific examples of saccharides that can be used in accordance with the present disclosure include glucose, fructose, sucrose, lactose, maltose, dextrin, cyclodextrin, and the like, alone or in combination. Also, a mixture of sucrose with corn syrup (containing glucose, maltose, and dextrin) can be used in accordance with this aspect of the present disclosure to produce a gas-containing effervescent agent. Other examples of compounds that are capable of being prepared in such a manner as to trap pressurized gas in cells include, for example, water soluble compounds such as salts, alkali halides, and alkaline earth metal halides. Specific salts useful in the present disclosure include, for example, sodium chloride, potassium chloride, potassium bromide, lithium chloride, cesium chloride, and the like. Typically, the cells containing the pressurized gas have a diameter of from about 5 micrometers to about 100 micrometers.

The substantially non-reactive gas can be infused into the cells of the soluble solid material to produce an effervescent agent useful in the present disclosure by first heating the starting material, such as a sugar, in a small amount of water until the material is dissolved. After dissolution of the material, the water is evaporated off leaving the material in a molten state. The molten material is then gasified by introducing a suitable gas, such as carbon dioxide, at a superatmospheric pressure into a sealed vessel containing the molten material. The molten material is agitated during gasification to ensure intimate contact between the molten material and the gas. Pressures of, for example, between about 50 psig (340 kPa) and about 1000 psig (6890 kPa) can be utilized to infuse the gas into the molten material. After gas infusion, the molten material is allowed to solidify while maintained in the sealed vessel to produce an effervescent agent. A suitable procedure of producing a gas-containing solid material is fully set forth in U.S. Pat. No. 4,289,794. The above procedure can produce solid effervescent agents containing cells of pressurized gas from about 50 psig (340 kPa) to about 900 psig (6200 kPa) which, when exposed to urine or other body exudates, allow the release of the trapped gas. This trapped gas, when released, can interact with the surfactant material in the container described herein. The container can suitably include from about 0.1 grams to about 15 grams of effervescent solid material containing a pressurized gas.

As noted above, the container additionally includes a surfactant. The surfactant component located in the permeable inflatable container is present as a foaming agent. When a gas, such as carbon dioxide, is produced from the gas generating system located in the container, the gas interacts with the surfactant and bubble-filled foam is produced. These bubbles inflate the container and cause it to swell and push against the bodyside liner 42 which, in turn, pushes against the skin of the wearer to alert the wearer to the nearing fullness of the absorbent body 44.

The surfactant used is not critical so long as it does not substantially irritate the skin upon contact. A wide variety of surfactants can be suitable for use in accordance with the present disclosure. For example, suitable surfactants include anionic surfactants, nonionic surfactants, amphoteric surfactants, cationic surfactants, and combinations thereof. Examples of suitable anionic surfactants include alkyl benzene sulfonates, alkyl sulfates, alkyl ether sulfates, sulfosuccinates, and combinations thereof. Examples of suitable nonionic surfactants include ethoxylated alcohols, fatty acid alkanolamides, ethoxylated alkanolamides, amine oxides, and combinations thereof. Examples of suitable amphoteric surfactants include alkyl betaines, amidobetaines, and combinations thereof. Examples of suitable cationic surfactants include alkylammonium halides. Generally, the container will include from about 0.1 grams to about 15 grams of surfactant.

In one aspect of the present disclosure, the components included in the system capable of generating a gas, such as carbon dioxide, upon being wetted and/or the surfactant present in the permeable inflatable container can be encapsulated in a urine-or-other-body-exudates-soluble shell material prior to introduction into the container. For example, if the system capable of generating a gas upon being wetted includes an acid and a base, the acid and the base can be separately encapsulated in a soluble encapsulation material to keep the components separated until wetted. Alternatively, the acid and base components can be encapsulated together if reactivity between the acid and the base in the absence of a liquid is not a concern. The surfactant can be separately encapsulated, or can be encapsulated with the acid and/or the base. Additionally, encapsulation can be used with gas-impregnated effervescent agents alone or in combination with the surfactant.

The shell material used for encapsulation can be suitably constructed of a material such that it will release the encapsulated material (i.e., the acid, base, effervescent agent and/or surfactant) upon contact with urine or other body exudates. The urine or other body exudates can cause the shell material to solubilize, disperse, swell, or disintegrate, or the shell material can be permeable such that it disintegrates or discharges the encapsulated material upon contact with urine or other body exudates. Suitable shell materials include cellulose-based polymeric materials (e.g., ethyl cellulose), carbohydrate-based materials (e.g., starches and sugars) and materials derived therefrom (e.g., dextrins and cyclodextrins) as well as other materials compatible with human tissues.

The shell thickness can vary depending upon the material encapsulated, and is generally manufactured to allow the encapsulated component to be covered by a thin layer of encapsulation material, which can be a monolayer or thicker laminate, or can be a composite layer. The layer should be thick enough to resist cracking or breaking of the shell during handling or shipping of the product, or during wear that could result in breakage of the encapsulating material. The material should also be constructed such that humidity from atmospheric conditions during storage, shipment, or wear will not cause a breakdown of the microencapsulation layer.

In another aspect of the present disclosure, a system similar to the pressure container described above can be used. Applying the appropriate substance(s) directly to or within the bodyside liner 42, absorbent body 44, or surge layer will result in a foaming or fizzing physical sensation upon insult with urine or other body exudates. In essence, the substances described above for use with an inflating container can be used without the container to produce a foaming or fizzing sensation directly to the wearer's skin. The substances are formed with or applied to the bodyside liner 42, absorbent body 44, or surge layer of the pants 20.

For example, the one or more of the substances described above could be combined in an air laid material or in a coform material and incorporated into the pants 20. As a specific example, tartaric acid can be combined with a coform on one layer with calcium carbonate on that or another layer. This material will then bubble vigorously when subjected to an aqueous solution. That bubbling is detectable to the wearer of the pants 20 and signals that the absorbent body 44 is nearing fullness.

Aspects of the disclosure have been described with reference to various specific and illustrative aspects and techniques. However, it should be understood that many variations and modifications can be made while remaining within the scope. Accordingly, this is intended to embrace all such alternatives, modifications and variations that fall within the scope of the appended claims. As various changes could be made in the above constructions and methods, without departing from the scope of the disclosure, it is intended that all matter contained in the above description and shown in the accompanying drawings shall be interpreted as illustrative and not in a limiting sense.

When introducing elements of the disclosure or the preferred aspect(s) thereof, the articles "a", "an", "the" and "said" are intended to mean that there are one or more of the elements. The terms "comprising," "including," and "having" are intended to be inclusive and mean that there can be additional elements other than the listed elements.

## Claims

1. An absorbent article for a wearer, the article comprising:
a liquid impermeable outercover;
an absorbent body disposed on the outercover; and
a physical sensation member disposed between the absorbent body and the wearer, wherein the physical sensation member is a temperature change material or a dimensional change member that provides a physical sensation to the wearer when contacted by a bodily exudate;
**characterised by** further comprising a liner disposed between the physical sensation member and the wearer, wherein the liner is attached to the outercover, the liner having a body-facing inner surface including body adhesive disposed thereon.

2. The article of claim 1, wherein the physical sensation is a cold sensation; or wherein the physical sensation is a warm sensation.

3. The article of claim 1, wherein the physical sensation is a pressure sensation.

4. The article of claim 1, wherein the physical sensation member includes a first carrier layer in superposed relationship with a physical sensation composite.

5. The article of claim 4. wherein the physical sensation member includes a second carrier layer, and wherein the first carrier layer and the second carrier layer sandwich the physical sensation composite.

6. The article of claim 5, wherein the first and second carrier layers are liquid permeable.

7. The article of claim 4, wherein the physical sensation composite includes temperature change material including an endothermic material.

8. The article of claim 7, wherein the physical sensation material is homogeneously intermixed within a matrix of fibers.

9. The article of claim 4, wherein the physical sensation composite includes temperature change material including an exothermic material.

## Patentansprüche

1. Saugfähiger Artikel für einen Träger, wobei der Artikel umfasst:
eine flüssigkeitsundurchlässige Außenhülle;
einen saugfähigen Körper, der auf der Außenhülle angeordnet ist; und
ein Körperempfindungselement, das zwischen dem saugfähigen Körper und dem Träger angeordnet ist, wobei das Körperempfindungselement ein Temperaturänderungsmaterial oder ein Abmessungsänderungsmaterial ist, das dem Träger ein Körperempfinden vermittelt, wenn es von einem Körperexsudat kontaktiert wird;
**dadurch gekennzeichnet, dass** es ferner eine Einlage umfasst, die zwischen dem Körperempfindungselement und dem Träger angeordnet ist, wobei die Einlage an der Außenhülle befestigt ist, wobei die Einlage eine dem Körper zugewandte Innenfläche enthält, auf der ein Körperhaftstoff angeordnet ist.

2. Artikel nach Anspruch 1, wobei das Körperempfinden ein Empfinden von Kälte ist; oder wobei das Körperempfinden ein Empfinden von Wärme ist.

3. Artikel nach Anspruch 1, wobei das Körperempfinden ein Empfinden von Druck ist.

4. Artikel nach Anspruch 1, wobei das Körperempfinden eine erste Trägerschicht in überlagertem Verhältnis mit einem Körperempfindungsverbundstoff enthält.

5. Artikel nach Anspruch 4, wobei das Körperempfindungselement eine zweite Trägerschicht enthält und wobei der Körperempfindungsverbundstoff zweiten der ersten Trägerschicht und der zweiten Trägerschicht angeordnet ist.

6. Artikel nach Anspruch 5, wobei die erste Trägerschicht und die zweite Trägerschicht flüssigkeitsdurchlässig sind.

7. Artikel nach Anspruch 4, wobei der Körperempfindungsverbundstoff ein Temperaturänderungsmaterial enthält, das ein endothermes Material enthält.

8. Artikel nach Anspruch 7, wobei das Körperempfindungsmaterial homogen in eine Fasermatrix eingemischt ist.

9. Artikel nach Anspruch 4, wobei der Körperempfindungsverbundstoff ein Temperaturänderungsmaterial enthält, das ein exothermes Material enthält.

## Revendications

1. Article absorbant pour un porteur, l'article comprenant :
une couche extérieure imperméable aux liquides ;
un corps absorbant placé sur la couche extérieure ; et
un élément de sensation physique placé entre le corps absorbant et le porteur, dans lequel l'élément de sensation physique est un matériau à changement de température ou un élément à changement dimensionnel qui fournit une sensation physique au porteur lorsqu'il est mis en contact avec un exsudat corporel ;
**caractérisé en ce qu'**il comprend en outre une doublure placée entre l'élément de sensation physique et le porteur, dans lequel la doublure est fixée à la couche extérieure, la doublure ayant une surface intérieure tournée vers le corps comprenant un adhésif corporel placé sur celle-ci.

2. Article selon la revendication 1, dans lequel la sensation physique est une sensation de froid ; ou dans lequel la sensation physique est une sensation de chaud.

3. Article selon la revendication 1, dans lequel la sensation physique est une sensation de pression.

4. Article selon la revendication 1, dans lequel l'élément de sensation physique comprend une première couche de support en relation de superposition avec un composite de sensation physique.

5. Article selon la revendication 4, dans lequel l'élément de sensation physique comprend une seconde couche de support et dans lequel la première couche de support et la seconde couche de support prennent en sandwich le composite de sensation physique.

6. Article selon la revendication 5, dans lequel les première et seconde couches de support sont perméables aux liquides.

7. Article selon la revendication 4, dans lequel le composite de sensation physique comprend un matériau à changement de température comprenant un matériau endothermique.

8. Article selon la revendication 7, dans lequel le matériau de sensation physique est mélangé de façon homogène au sein d'une matrice de fibres.

9. Article selon la revendication 4, dans lequel le composite de sensation physique comprend un matériau à changement de température comprenant un matériau exothermique.
